# EUROPEAN PATENT SPECIFICATION

(11) **EP 3 525 607 B1**
(45) Date of publication and mention of the grant of the patent: **02.12.2020**
(21) Application number: 17783972.7
(22) Date of filing: 04.10.2017
(51) Int. Cl.: A24F 47/00, A61M 15/06, A61M 11/04

(54) **AEROSOL PROVISION SYSTEM AND METHOD**
AEROSOLBEREITSTELLUNGSSYSTEM UND -VERFAHREN
SYSTÈME ET PROCÉDÉ DE FOURNITURE D'AÉROSOL

(30) Priority: 11.10.2016 GB 201617246
(43) Date of publication of application: 21.08.2019
(73) Proprietor: British American Tobacco (Investments) Limited, London WC2R 3LA (GB)
(72) Inventor: HEPWORTH, Richard, London WC2R 3LA (GB); DICKENS, Colin, London WC2R 3LA (GB); YUTERI, Caner, Southampton SO15 8TL (GB)
(74) Representative: D Young & Co LLP
(86) International application number: PCT/GB2017/052969
(87) International publication number: WO 2018/069673

(56) References cited:
- EP-A2- 2 797 448
- CN-A- 104 095 295
- FR-A1- 3 018 463
- US-A1- 2011 120 482
- US-A1- 2015 320 116
- US-A1- 2016 007 651

## Description

### Field

The present disclosure relates to an aerosol provision system and method.

### Background

Aerosol provision (AP) systems, such as e-cigarettes, non-combustion tobacco heating systems and other aerosol delivery systems, generally hold a payload that is either a reservoir of liquid which is to be vaporised, typically comprising nicotine (this is sometimes referred to as an "e-liquid"), or a reservoir of plant material or some other (ostensibly solid) plant derivative or material from which volatiles or other liquids or particulate solids may be liberated. When a user inhales on the device, typically an electrical (e.g. resistive) heater is activated to vaporise a small amount of liquid or release volatiles, particulates etc., in effect producing an aerosol which is consequently inhaled by the user. The liquid may comprise nicotine in a solvent, such as ethanol or water, together with glycerine or propylene glycol to aid aerosol formation, and may also include one or more additional flavours. The plant material may comprise tobacco or a derivative. The skilled person will be aware of many different payload formulations that may be used in AP systems.

The practice of inhaling an aerosol in this manner using such an AP system is commonly known as 'vaping'.

As a consequence, such AP systems are typically viewed as individual and personal items that it would be unhygienic to share with others, and which may also be perceived as becoming unhygienic over time by the system's owner, particularly if cleaning of the system is difficult. The present invention seeks to address or mitigate this problem.

Other previously proposed arrangements are disclosed in FR 3018463 and US 2011/120482.

### Summary

In a first aspect, an aerosol provision system is provided in accordance with claim 1.

In another aspect, a method of aerosol provision is provided in accordance with claim 11.

Further respective aspects and features of the invention are defined in the appended claims.

### Brief Description of the Drawings

Embodiments of the present invention will now be described by way of example with reference to the accompanying drawings, in which like reference numerals designate identical or corresponding parts throughout the several views:
- Figure 1 is a schematic diagram of an aerosol provision system in accordance with an embodiment of the present invention.
- Figures 2A-2C are illustrations of an aerosol provision system in accordance with an embodiment of the present invention.
- Figure 3 is a schematic diagram of an aerosol provision system in accordance with an embodiment of the present invention.
- Figure 4 is a schematic diagram of aerosol dispersion in an aerosol provision system in accordance with an embodiment of the present invention.
- Figure 5 is a flow diagram of a method of aerosol provision in accordance with an embodiment of the present invention.

### Detailed Description

An aerosol provision system and method are disclosed. In the following description, a number of specific details are presented in order to provide a thorough understanding of the embodiments of the present invention. It will be apparent, however, to a person skilled in the art that these specific details need not be employed to practice the present invention. Conversely, specific details known to the person skilled in the art are omitted for the purposes of clarity where appropriate.

As was noted previously herein, conventional AP systems such as e-cigarettes require the user to put the system in their mouth; this allows the user to draw in air and create an airflow through the device. Typically this airflow is detected and used to trigger heating of the liquid or solid payload to create an airborne payload (i.e. vapour, volatiles and/or particulates), which is then caught in the airflow to form an aerosolised payload that is inhaled by the user, although alternatively the device can be triggered via a button pressed substantially simultaneously to inhalation. It will be understood that where the description refers to 'liquid' or 'e-liquid' it encompasses equivalent solid plant matter sources, and similarly 'vapour' encompasses and equivalent volatiles and particulates (i.e. those contributing to an aerosolised payload for inhalation) unless explicitly stated.

Hence in such conventional systems, inhalation by the user is important to detect when to activate the system and to provide the airflow necessary to transport aerosolised payload to the user. However this requires that the aerosol provision system is held in the user's mouth sufficiently tightly that inhalation causes sufficient air to flow through the AP system.

As a result, it may be considered unhygienic to share an AP system between several users, and particularly between acquaintances and relative strangers such as may be encountered at a dinner party or other casual social gathering. This limits the scope for a communal experience and for sharing or trying out liquids and flavours enjoyed by others in the group.

Furthermore, even where the AP system is not intended to be shared, the use of the system may develop the perception that it becomes unhygienic over time, particularly if the mouthpiece is difficult to separate from the AP system and clean thoroughly.

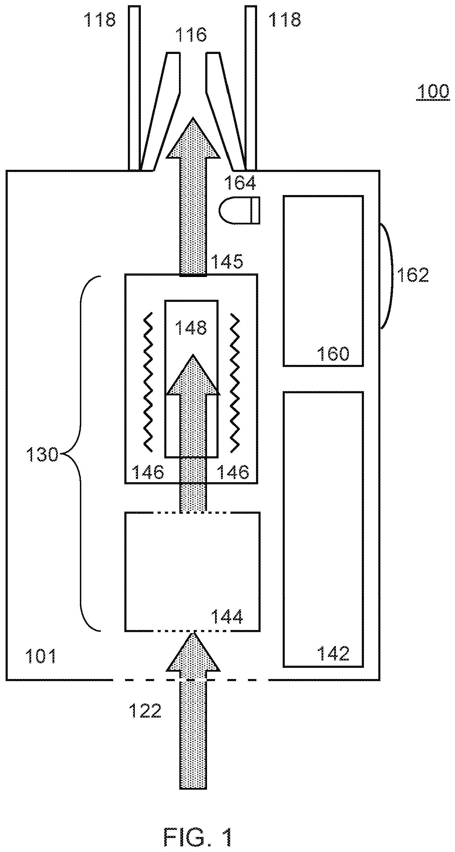

Accordingly, and referring now to Figure 1, in an embodiment of the present invention an AP system is arranged in operation to generate its own airflow, thereby removing the need for the user to generate the airflow themselves by forming a seal around the mouthpiece of the AP system with their mouth and inhaling.

The illustrated AP system 100 resembles a box-like case 101 with a nozzle 116 extending from the body of the base, although the particular shape of the case is not essential; for example a cylindrical case is also explicitly envisaged.

Inside the case, the AP system comprises a battery 142. The battery may be single use or rechargeable, and may or may not be accessible by the user. The AP system may also comprise a control unit to provide selective and/or regulated power to other components of the AP system.

The AP system also comprises an airflow generator 144 (for example a so-called micro-blower such as a piezoelectric blower or piezoelectric fan, or alternatively a motorised fan or pump; alternatively a compressed air source with an electrically actuated release may be used, but is not shown in the Figures), and also an atomiser 145, which includes the payload 148, e.g. a reservoir of liquid or solid material as described previously herein, and a heater 146. The heater illustrated in figure 1 is shown as a coil surrounding the payload, but this is a purely non-limiting example, and any suitable heating arrangement for generating vapour, volatiles or particulates as applicable from the payload (i.e. atomising a portion of the payload) is envisaged. The airflow generator 144 and the atomiser 145 together may be referred to as an aerosol generator 130.

Like a conventional AP system, the case 101 may provide access to the payload 148, which may take the form of a removable cartridge that can be replaced or interchanged with different cartridges to provide different flavours or strengths of vapour. Again such a cartridge may operate in a similar manner to cartridges in conventional AP systems. Alternatively, the payload may be inaccessible to the user; for example the AP system may be a disposable unit with a sealed case, whose operating life ends when the payload runs out.

In operation of the aerosol generator 130, the airflow generator 144 draws air in through one or more air vents 122 in the case and directs it to the atomiser 145. The or each vent can be positioned anywhere on the case that will enable suitable air flow to an intake of the airflow generator. Typically this will be on the underside of the case as held during normal use, as shown in Figure 1.

In general terms, the atomiser 145 operates in a similar manner to conventional AP systems, and may generate vapour in any suitable manner. For example, the payload 148 in the atomiser may hold an (e-)liquid directly in liquid form, or may utilise some absorbing structure, such as a foam matrix or cotton material, etc., as a retainer for the liquid. The liquid is then fed from the payload 148 to the heater 146 for atomisation (e.g. by vapourisation) to form an airborne payload. For example, the liquid may flow via capillary action from the payload 148 to the heater 146 via a wick (not shown in Figure 1). The air flow generated from the airflow generator then combines with the airborne payload to form an aerosol.

It will be appreciated that the aerosol may be formed without using a heater, such as via the use of piezo-electric vibration, or other mechanical means. It is also possible to create aerosols via electro-static atomisation, and the use of such in the present atomiser is explicitly contemplated. Hence the atomiser may employ any one or more of the above mechanisms to generate an aerosol, and references herein to a heater 146 incorporate these alternatives as applicable.

Similarly, as noted previously herein the liquid (or equivalently volatiles or particulates) may be provided in the form of plant material or some other (ostensibly solid) plant derivative or material. In this case the liquid can be considered as representing volatiles or particulates in the material which vaporise when the material is heated without combustion. Note that AP systems containing this type of material generally do not require a wick to transport the liquid to the heater, but rather provide a suitable arrangement of the heater in relation to the material to provide suitable heating.

In any event, the aerosol is then borne by the airflow out of the atomiser (and hence also the aerosol generator) and through the nozzle 116, optionally via flow guides or channels (not shown). The aerosol forms a distinct stream of aerosolised payload referred to herein as a 'ribbon', which then flows away from the nozzle. The ribbon may then be inhaled by the user without the need to physically place their mouth or nose in contact with the nozzle. An optional light such as an LED 164 can be provided to illuminate the ribbon.

In this way, the AP system provides the aerosolised payload in a manner that is more hygienic than a comparative device that requires the user to create a substantially hermetic seal around a mouthpiece with their mouth in order to draw air through the device by inhaling.

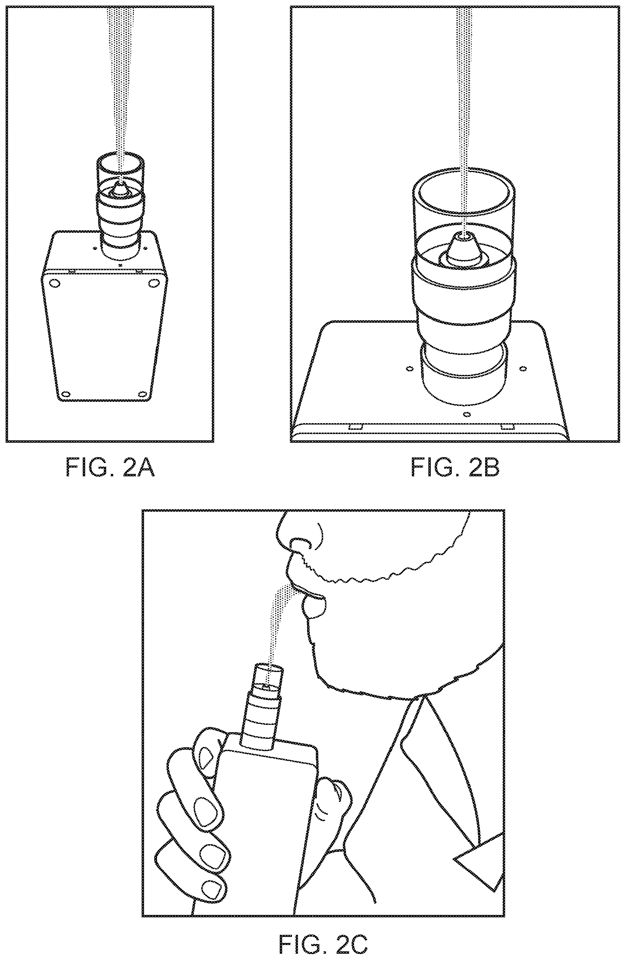

An exemplary illustration of the AP system in operation is shown in Figures 2A-2C. Figure 2A shows the AP system generating an aerosol stream or 'ribbon'. Figure 2B is a close-up of the ribbon flowing from the nozzle 116. Figure 2C illustrates how the ribbon may then be inhaled by a user without the need to place the device in their mouth (although they could do so if they wished, or could 'sip' from the nozzle, which is mounted external to the body of the case).

Because the present AP system does not require inhalation by the user to generate the airflow, and consequently cannot use detection of such an airflow to activate the atomiser (and airflow generator, in the case of the present AP system), an alternative control mechanism for generating aerosolised payload is required.

In an embodiment of the present invention, the AP system 100 consequently comprises a control button 162, activation of which may either directly supply power to the aerosol generator 130, or alternatively may provide a signal to the controller 160, which in turn provides appropriate power to the respective components of the aerosol generator, as will be described later herein.

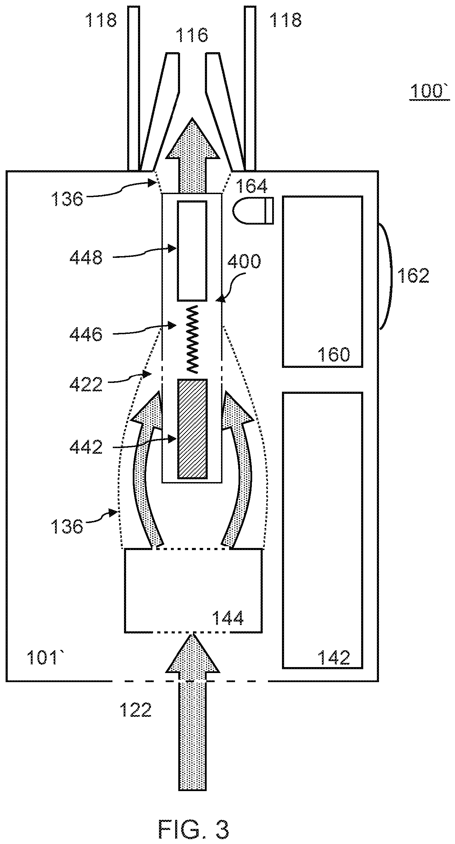

Referring now to Figure 3, in an alternative embodiment of the AP system 100', the atomiser 145 described previously is replaced by a separate AP system 400 in the form of a typical e-cigarette device or other suitable AP device that comprises its own battery 442, air intake vents 422, heater 446 and payload 448. Purely the purposes of clarity, the separate AP system 400 is referred to hereafter as an e-cigarette but it will be appreciated that this is purely illustrative and any suitable AP device may be considered.

In this alternative embodiment, the case 101' comprises a recess or receiver 136 into which the e-cigarette may be placed. The case still comprises an airflow generator 144 powered by the case battery 142, but this is now arranged to force or draw air through the e-cigarette in a manner similar to an inhalation by a user.

It will be appreciated that if the airflow generator is placed upstream of the e-cigarette (as illustrated in Figure 3), then it will force air through the e-cigarette and out through the nozzle, whereas if it is placed downstream of the e-cigarette it will draw air through the e-cigarette and out through the nozzle to a similar effect. Both arrangements are considered within the scope of the invention, although it will be noted that placing the airflow generator downstream of the e-cigarette may result in aerosolised payload condensing or otherwise accumulating on the moving parts of the airflow generator. This may not be a significant issue for a disposable device, but may affect functionality after prolonged use.

In any event, the e-cigarette responds to the generated air flow in a conventional fashion, by detecting a pressure drop due to airflow and activating its heater to create an aerosolised payload that is blown through the nozzle 116, to produce forms the distinct stream of aerosolised payload referred to herein as a 'ribbon', which then flows away from the nozzle.

The recess or receiver for the e-cigarette may have the dual role of acting as a flow guide to direct air emanating from the airflow generator towards air intake vents 422 of the e-cigarette, and to direct aerosolised payload forced or drawn from the separate AP system towards the nozzle 16.

Thus the alternative AP system 100' together with the e-cigarette 400 co-operate to function in a similar manner to the AP system of the preceding embodiment shown in Figure 1.

Advantageously this enables the conversion of conventional e-cigarettes and similar AP systems from inhalation-activated devices requiring mouth contact into streaming or ribbon devices from which aerosolised payload can be inhaled without the need for mouth contact.

The characteristics of the stream or ribbon of aerosolised vapour can be controlled in a number of ways. The characteristics of the ribbon potentially include its shape, its speed, its density and its frequency. Factors contributing to each of these are discussed below.

The shape of the ribbon is typically dependent upon the cross-section of the nozzle opening. A flat, letterbox-type opening would result in a planar ribbon of aerosolised payload, at least for a short distance from the AP system. Meanwhile a circular cross-section would result in a column of vapour as illustrated in figures 2A and 2B. Other nozzle opening shapes known in the art include but are not limited to a flat-fan, extended range flat-fan, even flat-fan, twin orifice flat-fan, hollow-cone and full-cone. It will be appreciated that nozzles could be interchangeable.

Optionally the shape may be changed dynamically, for example by utilising a mechanism of rotating discs in or beneath the body of a nozzle comprising a plurality of openings, in a manner similar to a showerhead mechanism. Consequently when using this mechanism, twisting the nozzle could change the effective cross-section of the ribbon from a wide stream to a narrow stream.

Similarly an elasticated nozzle with an electrically controlled actuator could change the effective cross-sectional area of the opening, as could a diaphragm shutter of the type found in cameras.

Similarly an electrically actuated needle or pin valve could be used in which a needle is mounted within the core of a tapered nozzle outlet; as the pin is moved closer to the nozzle outlet, the ribbon flow is cut off. Different needle profiles could be used to create different effects.

Similarly the airflow could be twisted as it exits the nozzle, for example by using two guide channels of different cross-sectional areas to supply the nozzle, resulting in a pressure differential in the cross-section of the combined aerosol, inter-resulting in a twisted airflow. Alternatively or in addition a rifled or corkscrew guide channel or inner surface of the nozzle could be provided.

Similarly, in conjunction with suitable control of airflow (described later herein) and for example by directing aerosolised vapour towards the walls of a guide channel or the nozzle, vortex rings could be produced by the AP system. Alternatively a ring-like aerosol distribution can be provided by a suitable nozzle such as a hollow cone nozzle or disk-and-core-cone nozzle.

It will be appreciated that one or more of the above shaping techniques can be used in conjunction with each other as appropriate; for example twisting the airflow may be performed in conjunction with adjustment of the nozzle size.

The speed of the aerosol stream forming the ribbon is responsive to the airflow generator pressure and the flow path.

The airflow generator pressure in turn will have maxima and minima determined by the choice of technology used to generate the airflow. Piezoelectric blower units and diaphragm pumps can for example generate a flow of 1 litre per minute, creating around 1.5-2.0kPa of static pressure (before any nozzle output). Meanwhile piezoelectric paddles act like a traditional handheld fan.

One or more such devices may be used in a single AP system, and so by way of a non-limiting example the flow rate may be in the order of 0.1 to 3 litres per minute, but more typically will be in the order of 1 litre per minute.

Notably piezoelectric devices such as those described above operate at a resonant frequency of the diaphragm or paddle; this is highly efficient, but generally means that the device operates at only one speed and only has one (maximum) flow rate. Typically however due to the size of these devices, and the material properties piezoelectric actuator, the resonant frequency is in the order of tens of thousands of Hertz. Therefore flow control can be achieved by use of a variable activation duty cycle operating in the order of tenths or hundredths of a second; the resulting rapid puffs of air rapidly blend and hence smooth out during their flow into and through the atomiser 145 or e-cigarette 400.

Furthermore in the case plural devices blowers or paddle being used, they could be selectively activated or deactivated to change the amount of airflow, although it will be appreciated that this could be relatively inefficient in terms of space, cost and power consumption.

Other technologies for the airflow generator include DC motor fans, which offer highly controllable airflow rates depending on the speed at which the fan is run. However, compared to piezoelectric pumps they are loud and have a relatively high power consumption. Furthermore they also comprise moving parts such as bearings that are subject to wear to tear, potentially resulting in a failure of the AP system or the need for servicing.

Finally, a compressed air canister with an electrically activated valve could be used. This may be suitable for example for a disposable device. Alternatively the canister could be recharged for example using a manual pump that may be integral to the case or provided separately. It will be appreciated that a compressed air canister could be used in conjunction with an electrically operated blower, paddle or fan to provide the occasional increase in airflow.

The airflow pressure generated by the airflow generator may consequently be controlled by controller (for example by use of duty cycles) to set a default level for the airflow properties of a particular AP system and/or nozzle setting as appropriate, in order to generate a ribbon of aerosolised payload with the desired properties. Consequently, the total airflow rate through the AP system may also be adjusted, as can the exit speed of the aerosolised payload from the nozzle, by increasing (if possible) or decreasing the default pressure level. This in turn can provide different properties relating to ribbon shape, ribbon extent and aerosolised payload density within the ribbon.

Optionally in addition the user could adjust the airflow rate, and consequently an input mechanism may be provided on the case of the AP system to perform this adjustment. For example 'speed up' and 'speed down' buttons or a dial may be provided in a manner similar to volume controls on a portable stereo. Hence from a default airflow rate setting, the controller could increase (if possible or decrease the airflow) in response to user input. The user can then see the effect this has on the property of the ribbon, and choose a speed setting they like. Optionally the controller can store this setting for subsequent use.

The aerosolised payload density is responsive to the airflow rate and the payload atomisation rate within the AP system. The atomisation rate in turn is typically a function of the amount of heat (or other atomising excitation) applied to the payload.

Again the level of heat or other excitation used to atomise the payload can be adjusted dynamically, for example by use of a duty cycle. In the case of a heater, the voltage/current applied to the heater can be adapted to produce more or less aerosolised payload (e.g. liquid vapour).

Consequently to maintain a desired density of aerosolised payload within the ribbon, the duty cycle or voltage/current used to control the rate of atomisation is preferably responsive to the flow rate through the atomiser.

Because the airflow is generated by known components and acts within a known flow path, it is possible to pre-calculate and consequently assume the flow rate for a given state of the airflow generator (and optionally also for a given state of the nozzle, where this is adjustable). Hence the control unit 160 may comprise a look up table associating control settings for the airflow generator with corresponding control settings for the heater or other excitation mechanism used to atomise the payload, so that as flow rate increases, the atomisation rate increases by a corresponding appropriate amount. The correspondence may be linear or non-linear and may be empirically determined.

Alternatively or in addition, the air flow rate through the atomiser may be directly measured in order to control the atomisation rate.

There are numerous techniques for measuring airflow that may be considered for use in the AP system, although some may either be too bulky, or expensive to be practical, or may provide either unnecessarily high accuracy or insufficient accuracy in the conditions provided by the system.

Airflow measurement options thus include:
- Differential pressure detectors; however, in practice the pressure drop caused by the airflow will be very low, and these detectors are also sensitive to changes in environmental temperature and pressure.
- Coriolis mass airflow detectors; such detectors are likely to have low accuracy in the flow ranges at issue within the AP system, and themselves result in a pressure drop that could affect the flow rate and ribbon characteristics of the AP system.
- Ultrasonic Doppler flow detectors; the accuracy of these detectors becomes low as the flow channel gets smaller, and rely on the aerosolised payload comprising particles large enough to cause reflection and of sufficient density to produce a measurable result, but beneficially these detectors do not interrupt the flow of the aerosol.
- Laser Doppler flow detectors; similar to ultrasonic Doppler flow detectors but can detect much smaller particles such as vapour particles, but may be relatively expensive.
- Thermal flow or 'hot wire' detectors; these measure the change in temperature on a hot wire or plate as air flows over it, taking away some heat by conduction. Such a sensor could be placed upstream of the heater in the atomiser so that it is independent of the heater's effects on the airstream. In principle, measurement of the heater itself could be used as a hot wire detector, using a measured difference between the expected and actual temperature of the heater as an indication of flow rate. In either case temperature can be detected by measuring the resistance of the hot wire or the heater, which will vary as a function of their temperature.
- Thermocouple detectors; these can be used to measure the temperature of the generated aerosol, with a comparative reduction in aerosol temperature indicating a higher flow rate. The comparison could be made against an assumed aerosol temperature for a given flow rate and a given heating temperature.
- Laminar flow detectors; these use differential flow monitoring of the outermost column of air touching the sides of a flow channel (and hence slowing down) versus the innermost column of air (being relatively frictionless). However such a device would be relatively invasive within the airflow, and also relatively expensive.

Hence whilst all of these detectors could be considered possible for use with the AP system, the most likely detector would be a hot wire detector, either as a separate hotwire anemometer within the flow path between the airflow generator and the atomiser, or derived from a measurement of the heater itself.

In any event, given either an assumed airflow rate and/or a measured airflow rate, the heater or excitation source in the atomiser can be adjusted to alter the density of aerosolised payload within the air by increasing or decreasing the heat/excitation.

Typically the density may be adjusted by the user, and consequently an input mechanism may be provided on the case of the AP system to perform this adjustment. For example 'density up' and 'density down' buttons or a dial may be provided in a manner similar to volume controls on a portable stereo. Hence from a default density setting, the controller 160 could increase or decrease the density of aerosolised payload within the ribbon in response to the user inputs. Optionally, the controller 160 could store an adjusted density setting for subsequent use. Also optionally, the controller 160 could impose a maximum density based upon the measured or known airflow rate.

It will be appreciated that in the embodiment where the atomiser was provided by a separate AP system such as an e-cigarette placed inside the case, the above-described density control may not be available. In this case, flow control may be used to provide an airflow rate calibrated to the particular type of e-cigarette in order to generate a default aerosol density from the e-cigarette. Alternatively however, e-cigarettes or other separate AP systems may be provided that are specifically compatible with the AP system, such that the AP system can control the heating / atomising behaviour of the separate AP system, for example via electrical contacts or wireless communication such as Bluetooth@, and the separate AP system may comprise a suitable flow sensor if flow measurement is used. In this way, aerosol density could be controlled using such a combination of devices.

Other control functions that may optionally be provided by the controller 160 include timing functions such as pre-heating the atomiser heater 146 a predetermined time before activating the airflow generator, so that atomised payload is being generated as the airflow reaches the atomiser 145 and can combine with it to generate aerosolised payload. Such a timing function may extend to time variant voltage/current control, for example to initially rapidly heat the heater to operating temperature before reducing voltage/current to a temperature maintaining level.

Timing may also be controlled to automatically turn off the airflow and heater after a predetermined period of time, in in order to control the total amount of aerosolised payload delivered in a single operation. This timing may override any manual control provided by the AP system (for example activation of button 162), or potentially vice versa. Such timing may also be used for other purposes, such as for example generating a pulsed ribbon, potentially comprising flows and gaps of different lengths to create patterns, or synchronising pulses with different coloured light from the optional light source 164.

As noted above, speed and aerosol density of the ribbon could be adjusted by the user, and these adjustments could be provided through controls on the AP device. Furthermore other control aspects such as timing could potentially be set or adjusted by the user. Consequently optionally these controls could be provided wirelessly as an alternative or in addition to controls on the AP system, for example via a Bluetooth@ link between the controller and a mobile phone or tablet running a controller app, or similarly using near field communication between the controller and the mobile phone or tablet.

Accordingly, it will be appreciated that the control methods discussed herein may be carried out on conventional hardware (such as the AP system and/or mobile phone as applicable) suitably adapted as applicable by software instruction or by the inclusion or substitution of dedicated hardware.

Thus the required adaptation to existing parts of a conventional equivalent device may be implemented in the form of a computer program product comprising processor implementable instructions stored on a non-transitory machine-readable medium such as a floppy disk, optical disk, hard disk, PROM, RAM, flash memory or any combination of these or other storage media, or realised in hardware as an ASIC (application specific integrated circuit) or an FPGA (field programmable gate array) or other configurable circuit suitable to use in adapting the conventional equivalent device. Separately, such a computer program may be transmitted via data signals on a network such as an Ethernet, a wireless network, the Internet, or any combination of these or other networks.

On the mobile phone or tablet, such software instruction may comprise implementing a method comprising the steps of:
- establishing communication (e.g. Bluetooth@ or NFC) with an AP system 100;
- optionally receiving status data from the AP system, such as for example present default values for heater temperature and airflow, from which an aerosol density value may be computed, and potentially other useful information such as battery charge level, payload level and the like;
- presenting to the user a user interface which may comprise controls for the AP system for one or more selected from the list consisting of:
   i. airflow rate generated by the AP system;
   ii. air speed at the nozzle;
   iii. heater temperature (for example as a min-max scale);
   iv. aerosolised payload density (for example as a 'strength' scale); and
   v. activation duration (for example as a min-max scale);
- reading one or more inputs from the user interface specifying one or more control values; and
- and then transmitting corresponding control values to the AP system, where necessary calculating appropriate control values, or optionally translating them into values appropriate for the AP system (for example translating a position on a min-max scale to a specific value responsive to the actual minimum and maximum values for the AP system).

Example calculations include calculating a heater temperature and airflow rate to achieve an indicated aerosolised payload density, as described previously herein, or calculating a suitable airflow to achieve the desired airspeed at the nozzle. Where the nozzle is interchangeable, the current nozzle configuration may be detected for example via the controller in the AP system, and transmitted to the mobile phone. Alternatively where the nozzle itself is configurable, a combination of airflow rate and nozzle configuration may be adjusted to achieve the desired airspeed.

Where the software is capable of supporting multiple AP system models, individual models may identify themselves during a handshaking process, and the relevant properties of the AP model can be retrieved by the software to define for example what controls may be available and presented to the user, and what ranges on those controls are available.

Correspondingly, on the AP system itself such software instruction may comprise implementing a method comprising the steps of:
- establishing communication (e.g. Bluetooth@ or NFC) with a remote device (e.g. a mobile phone or tablet);
- optionally transmitting status data to the remote device, such as for example present default values for heater temperature and airflow, from which an aerosol density value may be computed, and potentially other useful information such as battery charge level, payload level and the like;
- receiving from the remote device control values corresponding to the control of one or more selected from the list consisting of:
   i. airflow rate generated by the AP system;
   ii. air speed at the nozzle;
   iii. heater temperature;
   iv. aerosolised payload density; and
   v. activation duration;
- and then adjusting a behaviour of the or each respective component of the AP system for which a change of control value has been received.

Again, as with the mobile phone software, optionally if the mobile phone does not perform relevant calculations in response to certain user inputs such as a desired aerosolised payload density or strength of the ribbon, and instead simply transmits these user input values as proxies for control values, then these calculations can be performed by the controller 160 to obtain the correct control values.

As noted previously, a notable property of the ribbon is that it is intended for inhalation by its user preferably without the need to use a mouthpiece; consequently the AP system generates its own airflow. Probably therefore the user should be discouraged from placing the nozzle in their mouth, which may be done out of habit. Accordingly an optional nozzle guard 118 may be placed around the nozzle to deter or prevent the user from placing the nozzle in their mouth. Furthermore this guard may comprise one or more air vents near its base (i.e. near the end adjacent to the case 101)(not shown) so that if the user does place the nozzle guard in their mouth, it is still possible to maintain a pressure equilibrium. Such a guard may also serve to shield the nozzle outlet from disruptive external airflow that may affect the formation of the ribbon, such as wind. Figures 2A, 2B and 2C illustrate a transparent nozzle guard, by way of example only.

Another notable property of the ribbon is that it is intended for substantially immediate inhalation by a single user, and consequently it is generated for a short duration of time (typically in the order of 0.5 to 5 seconds) and in a tight stream emanating from the AP system nozzle.

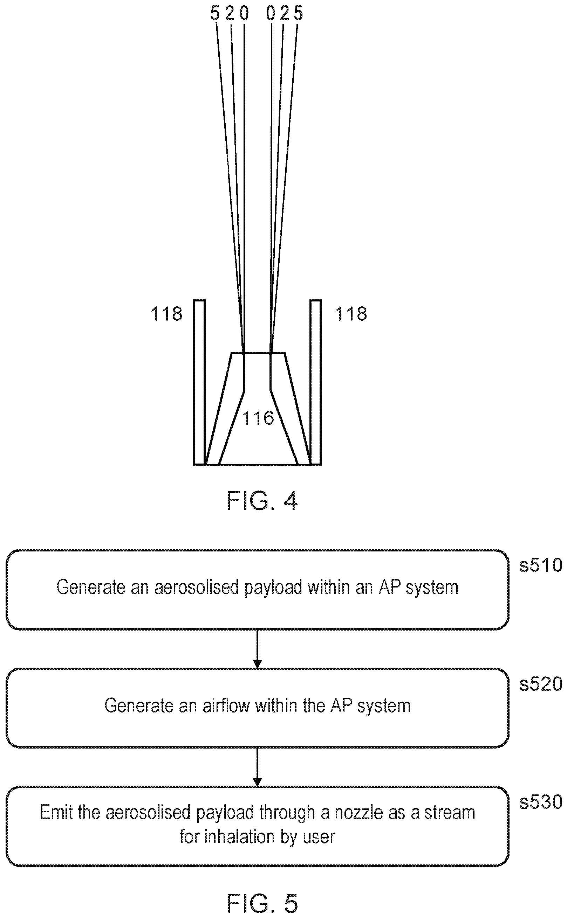

Referring now also to Figure 4, according to the present invention then in the absence of disruptive air flow (such as a transverse breeze, or user inhalation) this tight stream may be characterised as preferably having a dispersion angle in the range of 0-5° during the first 3cm beyond the nozzle, more preferably having a dispersion angle in the range 0-4° during the first 3 cm beyond the nozzle, more preferably having a dispersion angle in the range 0-3° during the first 3 cm beyond the nozzle, more preferably having a dispersion angle in the range 0-2° during the first 3 cm beyond the nozzle, and still more preferably having a dispersion angle in the range of 0-1° during the first 3 cm beyond the nozzle. The dispersion angle can be understood as characterising the change in width of the visible aerosol as a function of distance from the end of the nozzle. A purely parallel laminar flow would thus have a dispersion angle of 0°. Figure 4 illustrates selected dispersion angles of 5°, 2° and 0° for a notional 2.5mm diameter nozzle.

Meanwhile, Figures 2A and 2B illustrate a ribbon/stream having a dispersion angle of roughly 1° - 2° during the first 3 cm beyond the nozzle. Typically after this distance, air resistance and turbulence causes the stream to begin to break up.

The diameter of the nozzle outlet is typically in the order of 1-5 mm, and so consequently the diameter of the ribbon during the first 3 cm beyond the nozzle is similarly in the order of 1-5 mm.

A user may typically inhale from a distance of 1-15cm from the nozzle. Figure 2C illustrates a user inhaling at a distance of approximately 5cm.

Referring again to Figures 1 and 3, in a summary embodiment of the present invention an aerosol provision (AP) system (100, 100') comprises a power supply 142, an airflow generator 144 powered in operation by the power supply; and a nozzle 116. As explained previously herein, the airflow generator is arranged in operation to generate an airflow that passes firstly through an atomiser 145 to generate an aerosolised payload, and secondly through the nozzle; and the nozzle is arranged in operation to emit the aerosolised payload as a stream for inhalation by user.

It will be appreciated that consequently the user does not need to touch the aerosol provision system with their lips, although they can if they wish to do so. Hence optionally in an instance of this summary embodiment a mouthpiece may be provided that the user can removably attach to the nozzle in order to physically interact with the AP system in a more conventional manner by inhaling via the mouthpiece. This may be of use when weather conditions (e.g. wind) make use of the AP more difficult. Conversely, the user may wish to use such a mouthpiece by default, but has the option to remove it and make use of the ribbon/stream for example when relaxing at home, or when wishing to share the AP with friends.

In an instance of this summary embodiment, the AP system comprises the atomiser, for example as an integral or removable component of the AP system, and/or comprising an integral or replaceable payload. Optionally in this instance, the atomiser is part of a second separate AP system 400 (and hence is a removable component of the AP system 100'), and the AP system 100' comprises a receiver arranged to receive the second separate AP system 400. The receiver may be shaped to direct air flow generated by the airflow generator towards air inlets 422 of the second separate AP system, and/or to provide at least a partial seal downstream of the air inlets 422 of the second separate AP system, so as to force at least a proportion of the air to flow into the second separate AP system.

In an instance of this summary embodiment, the aerosolised payload is generated at a rate responsive to one or more selected from the list consisting of a predetermined airflow rate associated with the airflow generator (e.g. an assumed airflow rate for the present generator settings, as described previously herein), and a measurement of the airflow rate within a flow path of the air within the AP system (e.g. using any one or more of the flow rate sensors described previously herein). Optionally in this instance, the sensor is a thermal flow sensor. In this case, as described previously herein the heater of the atomiser can operate as a thermal flow sensor, for example by measuring its resistance as a function of temperature and comparing this with an expected resistance at an expected temperature for the given voltage/current driving the heater.

In an instance of this summary embodiment, the airflow generator comprises one or more selected from the list consisting of a piezoelectric blower, a piezoelectric paddle, a motorised fan, and a source of compressed air.

In an instance of this summary embodiment, the nozzle is removable from the AP system (for example by being unscrewed from the case) and hence is interchangeable with one or more alternative nozzles. Alternative nozzles can have different outlet shapes to change the shape and potentially the dispersion properties of the ribbon/stream, as discussed previously herein.

In an instance of this summary embodiment, the AP system comprises a nozzle guard arranged to prevent contact between the nozzle and a user's lips, for example if the user inadvertently attempts to inhale from the nozzle in a manner corresponding to the normal use of an inhalation activated AP system. As described previously herein, the nozzle may comprise vents so that inhalation by the user draws air in from the outside through the vents, and furthermore any airflow generated by the AP device has a means of exit in the event that the user does not inhale, thereby substantially avoiding any issue with pressure build-up within the device, which could strain the airflow generator or cause problems with aerosol deposition within the atomiser or other parts of the device.

In an instance of this summary embodiment, the AP system comprises a controller 160 and in response to user input, the controller is operable to change one or more selected from the list consisting of the airflow rate output by the airflow generator, and the aerosolised payload generation rate of the atomiser. As described previously herein, user input may be subject to maximum or minimum settings imposed by the controller, for example to constrain aerosolised payload density in the stream to within a preferred range. In this instance, controls could optionally be included in the AP system, for example in the form of buttons or dials providing input signals to the controller, alternatively or in addition the AP system can optionally comprise a wireless communication means such as Bluetooth ® or NFC communication means, and the wireless communication means receives the user input from a separate device (such as a mobile phone) comprising a corresponding wireless transmitter.

Turning now to Figure 5 in a summary embodiment of the present invention, a method of aerosol provision comprises:
- in a first step s510, generating an aerosolised payload within an AP system (100, 100'); and
- in a second step s520, generating an airflow within the AP system, wherein the generated air flow carries the aerosolised payload through a nozzle 116; and
- in a third step s530, emitting the aerosolised payload through a nozzle as a stream for inhalation by user. As noted previously, it will be appreciated that consequently the user does not need to touch the aerosol provision system with their lips, although they may wish to do so.

It will be appreciated that steps s510 and s520 may begin in any order, including simultaneously, and may substantially overlap. Similarly step s530 may substantially overlap with step s520 during operation.

In an instance of this summary embodiment, the step of generating an aerosolised payload within an AP system 100' is carried out by a second AP system 400 operably coupled to the AP system 100'.

In an instance of this summary embodiment, the step of generating an aerosolised payload comprises the step of generating the aerosolised payload at a rate responsive to one or more selected from the list consisting of a predetermined airflow rate associated with an airflow generator; and a measurement of the airflow rate within a flow path of the air.

In an instance of this summary embodiment, the method further comprises the step of providing a nozzle guard, arranged to prevent contact between the nozzle and a user's lips.

In a summary embodiment of the present invention, a computer readable medium having computer executable instructions adapted to cause a computer system to perform a method comprising the steps of establishing communication with an AP system 100 in accordance with claim 1, presenting to a user a user interface comprising one or more controls for the AP system for one or more selected from the list consisting of airflow rate generated by the AP system, air speed at the nozzle, heater temperature, aerosolised payload density, and activation duration; reading one or more inputs from the user interface specifying one or more control values; and transmitting corresponding control values to the AP system.

## Claims

1. A hand-held aerosol provision system (100, 100'), hereafter an AP system, comprising
a power supply (142);
an airflow generator (144) powered in operation by the power supply;
an atomiser (145);
a nozzle (116);
and wherein
the airflow generator is arranged in operation to generate an airflow that passes firstly through the atomiser (145) to generate an aerosolised payload, and secondly through the nozzle; and **characterized in that** the nozzle is arranged in operation to emit the aerosolised payload as a stream, having a dispersion angle in the range of 0-5° during the first 3cm beyond the nozzle, for inhalation by a user without the need to touch the aerosol provision system with their lips.

2. An AP system (100) according to claim 1, comprising the atomiser.

3. An AP system (100') according to claim 1, in which the AP system comprises a receiver arranged to receive a separate, second AP system (400); and
the atomiser is part of the second separate AP system.

4. An AP system in accordance with any one of the preceding claims, in which the aerosolised payload is generated at a rate responsive to one or more selected from the list consisting of:
i. a predetermined airflow rate associated with the airflow generator; and
ii. a measurement of the airflow rate within a flow path of the air within the AP system.

5. An AP system in accordance with any one of the preceding claims, in which the airflow generator comprises one or more selected from the list consisting of:
i. a piezoelectric blower;
ii. a piezoelectric paddle;
iii. a motorised fan; and
iv. a source of compressed air.

6. An AP system in accordance with any one of the preceding claims, in which the nozzle is removable from the AP system and interchangeable with one or more alternative nozzles.

7. An AP system in accordance with any one of the preceding claims, comprising:
a nozzle guard, arranged to prevent contact between the nozzle and a user's lips.

8. An AP system in accordance with claim 7, in which the nozzle guard is arrange to shield the nozzle outlet from disruptive external airflows.

9. An AP system in accordance with any one of the preceding claims, comprising:
a controller (160); and in which
in response to user input, the controller is operable to change one or more selected from the list consisting of:
i. the airflow rate output by the airflow generator; and
ii. the aerosolised payload generation rate of the atomiser.

10. An AP system in accordance with claim 9, comprising:
a wireless communication means; and in which
the wireless communication means receives the user input from a separate device comprising a wireless transmitter.

11. A method of aerosol provision, comprising the steps of:
generating an aerosolised payload within a hand-held aerosol provision system (100, 100'), hereafter an AP system; and
generating an airflow within the AP system;
wherein the generated air flow carries the aerosolised payload through a nozzle (116), and **characterized by** emitting the aerosolised payload through the nozzle as a stream, having a dispersion angle in the range of 0-5° during the first 3cm beyond the nozzle, for inhalation by user without the need to touch the aerosol provision system with their lips.

12. The method of aerosol provision of claim 11 the wherein the step of generating an aerosolised payload within an AP system (100') is carried out by a second AP system (400) operably coupled to the AP system.

13. The method of aerosol provision according to claim 11 or claim 12, wherein the step of generating an aerosolised payload comprises the step of
generating the aerosolised payload at a rate responsive to one or more selected from the list consisting of:
i. a predetermined airflow rate associated with an airflow generator; and
ii. a measurement of the airflow rate within a flow path of the air.

14. The method of aerosol provision according to any one of claims 11-13, comprising the step of:
providing a nozzle guard, arranged to prevent contact between the nozzle and a user's lips.

## Patentansprüche

1. Handgeführtes Aerosolbereitstellungssystem (100, 100'), nachfolgend ein AP-System, umfassend:
eine Stromversorgung (142);
einen Luftflussgenerator (144), der im Betrieb durch die Stromversorgung versorgt wird;
einen Zerstäuber (145);
eine Düse (116); und wobei
der Luftflussgenerator im Betrieb so angeordnet ist, dass er einen Luftfluss generiert, der erstens durch den Zerstäuber (145), um eine aerosolisierte Nutzlast zu generieren, und zweitens durch die Düse geleitet wird; und
**dadurch gekennzeichnet, dass**
die Düse im Betrieb so angeordnet ist, dass sie die aerosolisierte Nutzlast als Strom emittiert, der einen Dispersionswinkel im Bereich von 0 bis 5° während der ersten 3 cm hinter der Düse aufweist, um durch einen Benutzer inhaliert zu werden, ohne dass er das Aerosolbereitstellungssystem mit seinen Lippen berühren muss.

2. AP-System (100) nach Anspruch 1, umfassend den Zerstäuber.

3. AP-System (100') nach Anspruch 1, wobei das AP-System eine Annahme umfasst, die zum Annehmen eines separaten zweiten AP-Systems (400) angeordnet ist; und
wobei der Zerstäuber Teil des zweiten separaten AP-Systems ist.

4. AP-System nach einem der vorhergehenden Ansprüche, wobei die aerosolisierte Nutzlast mit einer Rate generiert wird, die auf ein oder mehrere ausgewählt aus der folgenden Liste anspricht, welche besteht aus:
i. einer vorbestimmten Luftflussrate, die zu dem Luftflussgenerator gehört; und
ii. einer Messung der Luftflussrate innerhalb eines Flusspfads der Luft innerhalb des AP-Systems.

5. AP-System nach einem der vorhergehenden Ansprüche, wobei der Luftflussgenerator ein oder mehrere ausgewählt aus der Liste umfasst, welche besteht aus:
i. einem piezoelektrischen Gebläse;
ii. einem piezoelektrischen Paddel;
iii. einem motorgetriebenen Ventilator; und
iv. einer Quelle für Druckluft.

6. AP-System nach einem der vorhergehenden Ansprüche, wobei die Düse von dem AP-System entfernbar und durch eine oder mehrere alternative Düsen austauschbar ist.

7. AP-System nach einem der vorhergehenden Ansprüche, umfassend:
einen Düsenschutz, der angeordnet ist, um Kontakt zwischen der Düse und den Lippen eines Benutzers zu verhindern.

8. AP-System nach Anspruch 7, wobei der Düsenschutz angeordnet ist, um den Düsenauslass gegen störende äußere Luftflüsse abzuschirmen.

9. AP-System nach einem der vorhergehenden Ansprüche, umfassend:
eine Steuerung (160); und wobei
die Steuerung funktional ist, um in Reaktion auf eine Benutzereingabe eine oder mehrere ausgewählt aus der Liste zu verändern, die besteht aus:
i. der Luftflussratenausgabe durch den Luftflussgenerator; und
ii. der Generierungsrate von aerosolisierter Nutzlast des Zerstäubers.

10. AP-System nach Anspruch 9, umfassend:
ein drahtloses Kommunikationsmittel; und wobei das drahtlose Kommunikationsmittel die Benutzereingabe von einer separaten Vorrichtung empfängt, die einen drahtlosen Sender umfasst.

11. Verfahren zur Aerosolbereitstellung, umfassend die Schritte:
Generieren einer aerosolisierten Nutzlast innerhalb eines handgeführten Aerosolbereitstellungssystems (100, 100'), nachfolgend eines AP-Systems; und
Generieren eines Luftflusses innerhalb des AP-Systems;
wobei der generierte Luftfluss die aerosolisierte Nutzlast durch eine Düse (116) trägt, und
**gekennzeichnet durch**:
Emittieren der aerosolisierte Nutzlast durch die Düse als Strom, der einen Dispersionswinkel im Bereich von 0 bis 5° während der ersten 3 cm hinter der Düse aufweist, um durch einen Benutzer inhaliert zu werden, ohne dass er das Aerosolbereitstellungssystem mit seinen Lippen berühren muss.

12. Verfahren zur Aerosolbereitstellung nach Anspruch 11, wobei der Schritt des Generierens einer aerosolisierten Nutzlast innerhalb eines AP-Systems (100') durch ein zweites AP-System (400) durchgeführt wird, das funktional an das AP-System gekoppelt ist.

13. Verfahren zur Aerosolbereitstellung nach Anspruch 11 oder Anspruch 12, wobei der Schritt des Generierens einer aerosolisierten Nutzlast den Schritt umfasst:
Generieren der aerosolisierten Nutzlast mit einer Rate, die auf eine oder mehrere ausgewählt aus der Liste anspricht, die besteht aus:
i. einer vorbestimmten Luftflussrate, die zu einem Luftflussgenerator gehört; und
ii. einer Messung der Luftflussrate innerhalb eines Flusspfads der Luft.

14. Verfahren zur Aerosolbereitstellung nach einem der Ansprüche 11 bis 13, welches die Schritte umfasst:
Bereitstellen eines Düsenschutzes, der angeordnet ist, um Kontakt zwischen der Düse und den Lippen eines Benutzers zu verhindern.

## Revendications

1. Système portatif de fourniture d'aérosol (100, 100'), ci-après système AP, comprenant
une source d'alimentation (142) ;
un générateur de flux d'air (144) alimenté en fonctionnement par la source d'alimentation ;
un atomiseur (145) ;
une buse (116) ;
et dans lequel
le générateur de flux d'air est conçu en fonctionnement pour générer un flux d'air qui passe tout d'abord à travers l'atomiseur (145) afin de générer une charge utile en aérosol, puis à travers la buse ;
et **caractérisé en ce que**
la buse est conçue en fonctionnement pour émettre la charge utile en aérosol sous la forme d'un courant, ayant un angle de dispersion dans la plage de 0 à 5° sur les 3 premiers centimètres au-delà de la buse, pour une inhalation par un utilisateur sans qu'il lui soit nécessaire de toucher le système de fourniture d'aérosol avec les lèvres.

2. Système AP (100) selon la revendication 1, comprenant l'atomiseur.

3. Système AP (100') selon la revendication 1, le système AP comprenant un récepteur conçu pour recevoir un second système AP distinct (400) ; et
l'atomiseur fait partie du second système AP distinct.

4. Système AP selon l'une quelconque des revendications précédentes, dans lequel la charge utile en aérosol est générée à un débit sensible à ou plusieurs parmi les éléments choisis dans la liste consistant en :
i. un débit d'air prédéfini associé au générateur de flux d'air ; et
ii. une mesure du débit d'air à l'intérieur d'un trajet d'écoulement de l'air dans le système AP.

5. Système AP selon l'une quelconque des revendications précédentes, dans lequel le générateur de flux d'air comprend un ou plusieurs éléments choisis dans la liste consistant en :
i. une soufflante piézoélectrique ;
ii. une palette piézoélectrique ;
iii. un ventilateur motorisé ; et
iv. une source d'air comprimé.

6. Système AP selon l'une quelconque des revendications précédentes, dans lequel la buse peut être retirée du système AP et peut être échangée avec une ou plusieurs autres buses.

7. Système AP selon l'une quelconque des revendications précédentes, comprenant :
une protection de buse, conçue pour éviter un contact entre la buse et les lèvres d'un utilisateur.

8. Système AP selon la revendication 7, dans lequel la protection de buse est conçue pour protéger la sortie de buse contre des flux d'air externes perturbateurs.

9. Système AP selon l'une quelconque des revendications précédentes, comprenant :
un organe de commande (160) ; et dans lequel en réponse à l'entrée utilisateur, l'organe de commande est exploitable pour changer un ou plusieurs éléments choisis dans la liste consistant en :
i. le débit d'air sorti par le générateur de flux d'air ; et
ii. le taux de génération de charge utile en aérosol de l'atomiseur.

10. Système AP selon la revendication 9, comprenant :
un moyen de communication sans fil ; et dans lequel
le moyen de communication sans fil reçoit l'entrée utilisateur en provenance d'un dispositif distinct comprenant un émetteur sans fil.

11. Procédé de fourniture d'aérosol, comprenant les étapes de :
génération d'une charge utile en aérosol à l'intérieur d'un système portatif de fourniture d'aérosol (100, 100'), ci-après système AP ; et
génération d'un flux d'air à l'intérieur du système AP ;
dans lequel le flux d'air généré transporte la charge utile en aérosol à travers une buse (116), et **caractérisé par**
l'émission de la charge utile en aérosol à travers la buse sous la forme d'un courant, ayant un angle de dispersion dans la plage de 0 à 5° sur les 3 premiers centimètres au-delà de la buse, pour une inhalation par un utilisateur sans qu'il lui soit nécessaire de toucher le système de fourniture d'aérosol avec les lèvres.

12. Procédé de fourniture d'aérosol selon la revendication 11, dans lequel l'étape de génération d'une charge utile en aérosol à l'intérieur d'un système AP (100') est réalisée par un second système AP (400) couplé fonctionnellement au système AP.

13. Procédé de fourniture d'aérosol selon la revendication 11 ou la revendication 12, dans lequel l'étape de génération d'une charge utile en aérosol comprend l'étape de
génération de la charge utile en aérosol à un débit sensible à ou plusieurs éléments choisis dans la liste consistant en :
i. un débit d'air prédéfini associé à un générateur de flux d'air ; et
ii. une mesure du débit d'air à l'intérieur d'un trajet d'écoulement de l'air.

14. Procédé de fourniture d'aérosol selon l'une quelconque des revendications 11 à 13, comprenant l'étape de :
fourniture d'une protection de buse, conçue pour éviter un contact entre la buse et les lèvres d'un utilisateur.
